# EUROPÄISCHE PATENTANMELDUNG

(11) **EP 1 759 727 A1**
(43) Veröffentlichungstag der Anmeldung: **07.03.2007**
(21) Anmeldenummer: 05019096.6
(22) Anmeldetag: 02.09.2005
(51) Int. Cl.: A61M 5/145

(54) **Infusionsgerät mit Steuereinheit**

(71) Anmelder: F.HOFFMANN-LA ROCHE AG, 4070 Basel (CH); Roche Diagnostics GmbH, 68305 Mannheim (DE)
(72) Erfinder: Huegli, Serge, 3422 Kirchberg (CH); De Polo, Marco, 4934 Madiswil (CH)
(74) Vertreter: Schwabe - Sandmair - Marx

(57) **Zusammenfassung**

Vorrichtung zur Verabreichung eines Produkts, die Vorrichtung umfassend:
a) ein Gehäuse (1, 2) mit einem Produktreservoir (4) oder einer Aufnahme (3) für ein Produktreservoir (4),
b) ein Abtriebsglied (7), das eine Abtriebsbewegung in eine Vortriebsrichtung (A) ausführt, um Produkt aus dem Produktreservoir (4) auszuschütten, und das gegen die Vortriebsrichtung (A) eine Rückwärtsbewegung bis in eine hintere Position ausführen kann,
c) ein von dem Gehäuse (1, 2) zur Ausführung einer Antriebsbewegung bewegbar gelagertes Antriebsglied (10), das mit dem Abtriebsglied (7) so gekoppelt ist, dass die Antriebsbewegung die Abtriebsbewegung bewirkt und das Abtriebsglied (7) sich bei der Abtriebsbewegung an dem Antriebsglied (10) gegen die Vortriebsrichtung (A) abstützt,
d) eine Überwachungseinrichtung mit einer Ausgabeeinrichtung (22) zur Ausgabe eines Alarms und/oder einer Steuerung (21) für einen Antrieb (20) des Antriebsglieds (10)
e) und einen Sensor (16, 17), der bei der Rückwärtsbewegung des Abtriebsglieds (7) das Erreichen der hinteren Position detektiert und an die Überwachungseinrichtung ein für das Erreichen der hinteren Position charakteristisches Ausgangssignal ausgibt.

## Beschreibung

Die Erfindung betrifft eine Vorrichtung zur Verabreichung eines Produkts in medizinischen, pharmazeutischen und kosmetischen Applikationen. Vorzugsweise handelt es sich um eine Vorrichtung für die Selbstverabreichung des Produkts, beispielsweise von Insulin in der Diabetestherapie. Die Vorrichtung kann insbesondere ein Infusionsgerät sein.

Solche Vorrichtungen weisen eine Fördereinrichtung mit einem Antriebsglied und einem Abtriebsglied auf, die so miteinander gekoppelt sind, dass eine Antriebsbewegung des Antriebsglieds eine Abtriebsbewegung des Abtriebsglieds bewirkt. Durch die Abtriebsbewegung des Abtriebsglieds wird Produkt aus einem Produktreservoir der Vorrichtung ausgeschüttet und verabreicht, meist subkutan. Bei üblichen Fördereinrichtungen, beispielsweise Kolbenfördereinrichtungen, führt das Abtriebsglied die Abtriebsbewegung bis in eine vordere Endposition aus, die es nach Entleerung des Reservoirs einnimmt. Für den Austausch oder das Auffüllen des Reservoirs muss das Abtriebsglied aus der vorderen Endposition wieder in die hintere Endposition zurückbewegt werden. Um die hintere Endposition zu definieren, bildet das Antriebsglied üblicherweise einen Anschlag, gegen den das Abtriebsglied zurückbewegt wird. Die Justierung des Anschlags ist allerdings schwierig. Wird das Antriebsglied von einem Elektromotor angetrieben, wird das Erreichen der hinteren Endposition, nämlich der Druck des Abtriebsglieds gegen den Anschlag, durch die Steigerung der Leistungsaufnahme des Motors festgestellt. Wird das Abtriebsglied manuell in die hintere Endposition zurückbewegt, muss der Verwender selbst den erhöhten Widerstand spüren. In beiden Fällen besteht die Gefahr, dass die Fördereinrichtung durch das Andrücken des Abtriebsglieds beschädigt wird.

Es ist eine Aufgabe der Erfindung, eine Produktverabreichungsvorrichtung mit einer Fördereinrichtung zu schaffen, die für den Austausch oder das Auffüllen eines Produktreservoirs sicher in einen wohldefinierten Ausgangszustand gebracht werden kann.

Die erfindungsgemäße Vorrichtung umfasst ein Gehäuse mit einer Aufnahme für ein Produktreservoir in Form eines in der Aufnahme aufnehmbaren Behältnisses, vorzugsweise eine Ampulle. Grundsätzlich möglich ist es jedoch auch, dass das Gehäuse das Reservoir unmittelbar bildet. Die Vorrichtung umfasst ferner eine Fördereinrichtung, mittels der das Produkt aus dem Reservoir förderbar ist. Die Fördereinrichtung kann insbesondere einen in dem Reservoir aufgenommenen Kolben umfassen. Auf jeden Fall umfasst die Fördereinrichtung ein Abtriebsglied und ein Antriebsglied. Um Produkt aus dem Reservoir auszuschütten, führt das Abtriebsglied eine Abtriebsbewegung in eine Vortriebsrichtung aus. Für einen Austausch oder ein Auffüllen des Reservoirs führt es eine Rückwärtsbewegung gegen die Vortriebsrichtung bis in eine hintere Position aus. Das Antriebsglied wird von dem Gehäuse zur Ausführung einer Antriebsbewegung bewegbar gelagert. Das Antriebsglied und das Abtriebsglied sind so miteinander gekoppelt, dass die Antriebsbewegung des Antriebsglieds die Abtriebsbewegung des Abtriebsglieds bewirkt und das Abtriebsglied sich bei der Abtriebsbewegung an dem Antriebsglied gegen die Vortriebsrichtung abstützt. Die Kopplung ist mechanisch. Vorzugsweise sind das Antriebsglied und das Abtriebsglied mittels eines Gewindeeingriffs dieser beiden Glieder unmittelbar oder über ein oder mehrere Zwischenglieder miteinander gekoppelt. Die Abtriebsbewegung und die Rückwärtsbewegung sind einander entgegengerichtete Bewegungen, vorzugsweise lineare Translationsbewegungen, entlang einer Translationsachse. Die Antriebsbewegung ist vorzugsweise eine reine Rotationsbewegung um eine Rotationsachse. In besonders bevorzugten Ausführungen fallen die Translationsachse und die Rotationsachse zusammen.

Die Vorrichtung umfasst ferner eine Überwachungseinrichtung mit einer Ausgabeeinrichtung zur Ausgabe eines Alarms oder einer Steuerung für einen Antrieb des Antriebsglieds, falls ein solcher vorhanden ist, wie dies in den bevorzugten Ausführungen der Vorrichtung als Infusionsgerät der Fall ist. Insbesondere kann ein Elektromotor, beispielsweise ein Schrittmotor, solch einen Antrieb bilden. Ist ein Fremdantrieb nicht vorhanden, so dass das Antriebsglied manuell angetrieben wird, wie dies beispielsweise bei den üblichen Injektionsgeräten der Fall ist, so weist die Überwachungseinrichtung lediglich die Ausgabeeinrichtung auf. In jedem Fall umfasst die Überwachungseinrichtung wenigstens eines aus a) Ausgabeeinrichtung und b) Antrieb mit Steuerung, vorzugsweise umfasst sie beide Einrichtungen. Die Ausgabeeinrichtung kann ausschließlich eine optische Ausgabe, ausschließlich eine akustische Ausgabe oder auch nur eine taktile Ausgabe aufweisen, vorzugsweise weist sie jedoch eine Kombination aus wenigstens zwei der genannten Ausgaben auf. Die Bezeichnung "Alarm" wird in einem weiten Sinne verstanden. Der Alarm kann zwar insbesondere bei Ausgabe eines akustischen oder taktilen Alarmsignals oder bei optischer Ausgabe mittels eines Blinksignals als solcher besonders kenntlich gemacht sein. Grundsätzlich wird als Alarm jedoch auch eine beispielsweise schlichte optische Anzeige verstanden.

Nach der Erfindung umfasst die Vorrichtung auch einen Sensor, der im Falle einer Rückwärtsbewegung des Abtriebsglieds das Erreichen der hinteren Position detektiert und an die Überwachungseinrichtung ein für das Erreichen der hinteren Position charakteristisches Ausgangssignal ausgibt und so das Erreichen der hinteren Position "meldet". Der Sensor kann zwar grundsätzlich ein berührungslos arbeitender Sensor sein, beispielsweise ein optischer Positionssensor. In bevorzugten Ausführungen ist der Sensor jedoch ein Berührungssensor. Der Berührungssensor kann beispielsweise ganz einfach nur feststellen, dass er Anschlagkontakt mit dem Abtriebsglied hat. Bevorzugt ist der Berührungssensor jedoch ein Kraftsensor, der unmittelbar die Kraft, mit der das Abtriebsglied gegen den Sensor drückt, oder eine andere Größe misst, aus der auf diese Kraft geschlossen werden kann. Solch eine Kraftsensor kann insbesondere mittels Dehnungsmesssstreifen (DMS) gebildet sein.

Falls die Vorrichtung über einen motorischen Antrieb verfügt, wie dies bevorzugt wird, kann das Ausgangssignal des Sensors vorteilhafterweise der Antriebssteuerung dazu dienen, den Antrieb festzusetzen, vorzugsweise abzuschalten.

Indem bei dem Abtriebsglied das Erreichen der hinteren Position mittels eines Sensors detektiert und dessen Ausgangssignal an die Überwachungseinrichtung weitergeleitet wird, ist das Erreichen der hinteren Position früher als bei den herkömmlichen Vorrichtungen feststellbar. Hierdurch wird die Gefahr von Beschädigungen der Fördereinrichtung, beispielsweise innerhalb der Kopplung von Antriebs- und Abtriebsglied oder eines vorzugsweise vorhandenen Antriebsmotors verringert. Des Weiteren muss auch kein Festanschlag exakt und daher aufwendig montiert werden.

In bevorzugten Ausführungen misst der Sensor eine Lagerkraft, die das Gehäuse aufnimmt, wenn das Abtriebsglied die Abtriebsbewegung ausführt. Hierfür ist es vorteilhaft, wenn das Antriebsglied sich gegen die Vortriebsrichtung an dem Sensor abstützt. Solch eine Abstützung des Antriebsglieds ist beispielsweise aus der US-PS-5,647,853 bekannt. Die Abstützung der kompletten Fördereinrichtung an einem Kraftsensor geht aus der US-PS-6,368,314 hervor. In den bekannten Vorrichtungen dient der Sensor jedoch ausschließlich der Detektion von Okklusionen und Lecks in einer der das Produkt führenden Komponenten der betreffenden Vorrichtung. In den Ausführungen der Erfindung, in denen der Sensor die aus der Abtriebsbewegung des Abtriebsglieds resultierende, letztlich vom Gehäuse aufzunehmende Lagerkraft misst, erfüllt der Sensor, vorzugsweise Kraftsensor, der Erfindung in den bevorzugten Ausführungen jedoch eine Doppelfunktion, nämlich die Funktionen der Messung der Lagerkraft und der Detektion der Position des Abtriebsglieds in dessen hinteren Position. Die erfindungsgemäße, neue Funktion des Sensors hat auch positive Rückwirkungen auf die bereits bekannte Funktion der Lagerkraftmessung. Wird nämlich mittels des Sensors für die Messung der Lagerkraft gleichzeitig auch das Erreichen der hinteren Position detektiert, so kann mittels der erfindungsgemäßen Funktion auf einfache Weise die Funktionstüchtigkeit des Okklusions- und/oder Lecksensors geprüft werden. Detektiert der Sensor das Erreichen der hinteren Position, so kann mit ausreichender Sicherheit dessen Funktionstüchtigkeit für die Funktion der Okklusions- und/oder Leckdetektion unterstellt werden.

Das Antriebsglied ist bei der Abtriebsbewegung des Abtriebsglieds vorzugsweise über einen relativ zu dem Antriebsglied beweglichen Übertragungskörper an dem Sensor abgestützt. Der Sensor drückt den Übertragungskörper zumindest während der Abtriebsbewegung in die Vortriebsrichtung gegen einen Anschlag des Antriebsglieds. Die Kraftübertragung zwischen dem Antriebsglied und dem Sensor mittels des Übertragungskörpers gestattet vorteilhafterweise die Entkopplung des Sensors von der Antriebsbewegung des Antriebsglieds, indem das Antriebsglied die Antriebsbewegung relativ zu dem Übertragungskörper ausführt.

Vorteilhafterweise ist das Antriebsglied in und gegen die Vortriebsrichtung schwimmend gelagert und wird vorzugsweise gegen die Vortriebsrichtung von dem Sensor elastisch abgestützt und von einer von dem Sensor ausgeübten Elastizitätskraft in die Vortriebsrichtung gegen einen Anschlag des Gehäuses gedrückt. Die schwimmende Lagerung kann mittels des elastisch nachgiebigen Sensors vorteilhafterweise spielfrei gebildet werden.

Bevorzugte Merkmale der Erfindung werden auch in den Unteransprüchen und durch die Kombinationen der Ansprüche offenbart.

Nachfolgend wird ein bevorzugtes Ausführungsbeispiel der Erfindung anhand einer Figur erläutert. An dem Ausführungsbeispiel offenbar werdende Merkmale bilden je einzeln und in jeder Merkmalskombination die Gegenstände der Ansprüche und die vorstehend beschriebenen Ausgestaltungen vorteilhaft weiter.

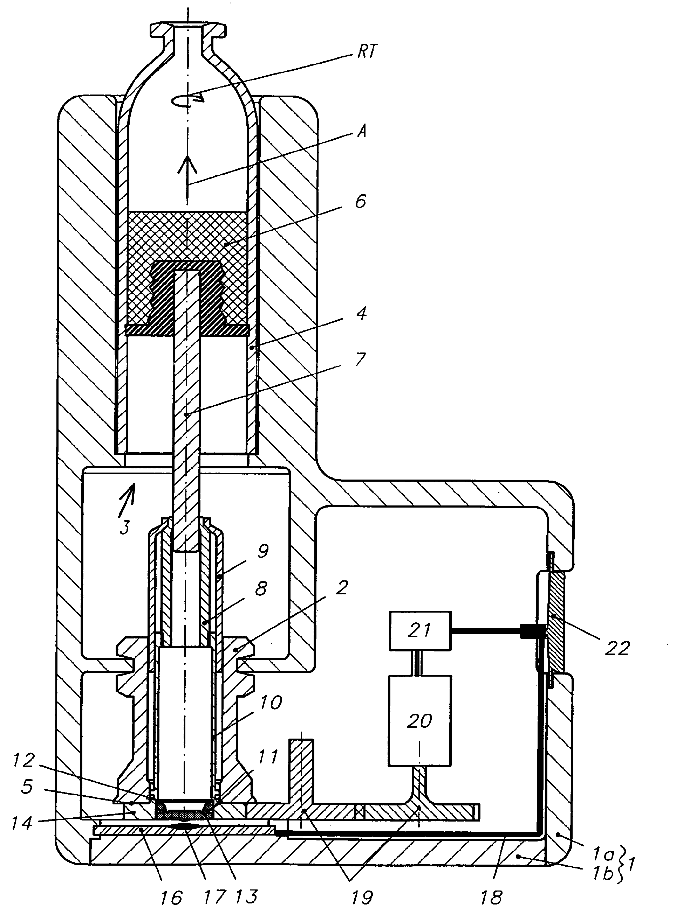

Die Figur zeigt als Beispiel einer Produktverabreichungsvorrichtung ein bequem unter oder auf der Kleidung tragbares Infusionsgerät für die Selbstverabreichung eines flüssigen Medikaments, beispielsweise für die Verabreichung von Insulin in der Diabetestherapie. Das Infusionsgerät umfasst ein Gehäuse mit einer Hüllstruktur 1 und einer Lagerstruktur 2, die in der Hüllstruktur 1 nicht bewegbar aufgenommen ist und der Lagerung eines Teils einer Fördereinrichtung dient. Die Hüllstruktur 1 umfasst ein Hauptteil 1a und einen damit fest verbundenen Verschlussdeckel 1b. Die Hüllstruktur 1 bildet zum Einen die Aufnahme für die Fördereinrichtung und zum anderen eine Aufnahme 3, in der ein Reservoir 4 für das Medikament aufgenommen ist. Das Reservoir 4 wird im Ausführungsbeispiel von einer Ampulle gebildet, in der ein Kolben 6 in eine Vortriebsrichtung A gleitverschiebbar aufgenommen ist. Durch Verschiebung des Kolbens 6 in die Vortriebsrichtung A wird Produkt aus dem Reservoir 4 ausgeschüttet und durch einen an den Auslass des Reservoirs angeschlossenen Katheter verabreicht. Das Reservoir 4 ist in der Aufnahme 3 in einer definierten Position und Ausrichtung aufgenommen.

Die Fördereinrichtung umfasst ein Abtriebsglied 7, im Ausführungsbeispiel eine mit dem Kolben 6 verbundene Kolbenstange. Die Fördereinrichtung umfasst ferner ein Antriebsglied 10, das von der Lagerstruktur 2 um eine Rotationsachse RT drehgelagert wird, ein Zwischenglied 8 und eine Verdrehsicherung 9 für das Abtriebsglied 7. Das Antriebsglied 10 ist über das Zwischenglied 8 mechanisch mit dem Abtriebsglied 7 gekoppelt. Das Antriebsglied 10, das Zwischenglied 8 und das Abtriebsglied 7 bilden je eine Stufe eines Teleskopantriebs. Das Antriebsglied 10 ist mit dem Zwischenglied 8 in einem ersten Gewindeeingriff, und das Zwischenglied 8 ist mit dem Abtriebsglied 7 in einem zweiten Gewindeeingriff. Auf diese Weise werden zwei Spindeltriebe erhalten. Das Abtriebsglied 7 ist hierfür über einen großen Teil seiner axialen Länge mit einem Außengewinde versehen. Das Zwischenglied 8 ist ein Hülsenbauteil, das das Abtriebsglied 7 konzentrisch zur Rotationsachse RT umgibt und an seinem in Vortriebsrichtung A vorderen Ende ein Innengewinde aufweist, das mit dem Außengewinde des Abtriebsglieds 7 in dem zweiten Gewindeeingriff ist. Das Zwischenglied 8 ist ebenfalls über einen großen Teil seiner axialen Länge mit einem Außengewinde versehen. Das Antriebsglied 10 weist an seinem vorderen Ende ein Innengewinde auf, das mit dem Außengewinde des Zwischenglieds 8 in dem ersten Gewindeeingriff ist. Die Lagerstruktur 2 führt die Verdrehsicherung 9 axial linear. Die Verdrehsicherung 9 führt ihrerseits das Abtriebsglied 7 axial linear. Das Zwischenglied 8 nimmt die Verdrehsicherung 9 bei Bewegungen in und gegen die Vortriebsrichtung A mit.

Das Antriebsglied 10 ist an seinem hinteren Ende umlaufend mit einem außenverzahnten Ringsteg 14 versehen, über den das Antriebsglied 10 um die Rotationsachse RT drehangetrieben wird. Der Antrieb 20 ist ein Drehantrieb, der über ein Untersetzungsgetriebe 19 das Antriebsglied 10 drehantreibt, indem ein Ausgangsrad des Untersetzungsgetriebes 19 mit dem Ringsteg 14 in einem Zahneingriff ist. Für den Drehantrieb sorgen ein in der Hüllstruktur 1 angeordneter Antrieb 20, im Ausführungsbeispiel ein elektrischer Schrittmotor, den eine Antriebssteuerung 21 positionsgenau steuert. Die Antriebssteuerung 21 ist Bestandteil einer Überwachungseinrichtung, die auch eine Ausgabeeinrichtung 22 und eine nicht dargestellte Eingabeeinrichtung umfasst. Die Ausgabeeinrichtung 22 ist eine optische Ausgabe mit einem Display, auf dem relevante Daten des Geräts und auch der Produktverabreichung, beispielsweise eine Verabreichungshistorie, angezeigt werden oder zumindest anzeigbar sind.

Die Hüllstruktur 1 lagert ferner einen Kraftsensor. Der Kraftsensor umfasst einen gegen und vorzugsweise auch in die Vortriebsrichtung A elastisch nachgiebigen Körper 16, den die Hüllstrukur 1 an wenigstens zwei Stellen fest eingespannt hält. Der Körper 16 ist formelastisch. Im Ausführungsbeispiel bildet er zwischen den beiden Einspannstellen einen elastischen Biegebalken und wird im Folgenden deshalb als Biegekörper bezeichnet. Der Biegekörper 16 dient als Träger für wenigstens einen Dehnmessstreifen oder bildet selbst einen Dehnmessstreifen. Zentral zwischen den beiden Einspannstellen ragt an der in die Vortriebsrichtung A weisenden Vorderseite des Biegekörpers 16 eine buckelförmige Erhebung 17 ab. Der so gebildete Sensor 16, 17 ist mittels einer Signalleitung 18 mit der Antriebssteuerung 21 und der Ausgabeeinrichtung 22 verbunden. Über die Signalleitung 18 wird den beiden Einrichtungen 21 und 22 je das gleiche Ausgangssignal des Sensors 16, 17 zugeführt.

Das Antriebsglied 10 ist schwimmend auf dem Sensor 16, 17 gelagert. Hierfür ist das Antriebsglied 10 relativ zu der Lagerstruktur 2 und damit auch relativ zu der Hüllstruktur 1 axial beweglich gelagert und wird von der Lagerstruktur 2 axial linear gleitend geführt. Ein an der inneren Mantelfläche der Lagerstruktur 2 umlaufender Ringsteg 5 bildet einen Teil der Gleitführung und begrenzt die axiale Beweglichkeit des Antriebsglieds 10 in die Vortriebsrichtung A. Hierfür bildet die Lagerstruktur 2 mit dem Ringsteg 5 einen gegen die Vortriebsrichtung A weisenden Anschlag für das Antriebsglied 10. Das Antriebsglied 10 ist so montiert, dass der Biegekörper 16 mit einer bestimmten, in die Vortriebsrichtung A wirkenden Elastizitätskraft gegen das Antriebsglied 10 und das Antriebsglied 10 dabei gegen den vom Ringsteg 5 gebildeten Anschlag drückt. Die Überwachungseinrichtung ist so eingestellt, dass diese bestimmte, vorgegebene Elastizitätskraft des Biegekörpers 16 als Kraftniveau "null" angenommen wird. Die Dimensionierung des Sensors 16, 17 und dessen Anordnung sowie die Anordnung des Antriebsglieds 10 sind ferner so, dass das Antriebsglied 10 zumindest in jeder normalen Betriebssituation von dem Sensor 16, 17 gegen den vom Ringsteg 5 gebildeten Anschlag gedrückt wird. Anzumerken ist noch, dass über die Kopplung mittels des Zwischenglieds 8 letztlich auch das Abtriebsglied 7 zusammen mit dem Antriebsglied 10 schwimmend gelagert ist. Falls der Kolben 6 mit dem Abtriebsglied 7 axial nicht beweglich verbunden ist, ist letztlich auch der Kolben 6 mit dem Antriebsglied 10 schwimmend gelagert. Der Kolben 6 kann stattdessen auch losgelöst von dem Abtriebsglied 7 in dem Reservoir 4 aufgenommen sein, und das Abtriebsglied 7 kann in die Vortriebsrichtung A lose gegen die freie Rückseite eines solchen Kolbens drücken.

Um den Sensor 16, 17 von der Antriebsbewegung des Antriebsglieds 10 zu entkoppeln, d. h. freizuhalten, stützt sich das Antriebsglied 10 nicht unmittelbar selbst an dem Sensor 16, 17 ab, sondern erst über einen Übertragungskörper 13. Der Übertragungskörper 13 ist relativ zu dem Antriebsglied 10 in und gegen die Vortriebsrichtung A beweglich, wobei die Bewegung in die Vortriebsrichtung A von einem Anschlag 12 des Antriebsglieds 10 begrenzt wird. Der Sensor 16, 17 drückt mit seiner Elastizitätskraft in die Vortriebsrichtung A gegen den Übertragungskörper 13, der seinerseits gegen den Anschlag 12 drückt. Der Lagerkörper 13 ist relativ zu dem Antriebsglied 10 nicht nur axial, sondern auch um die Rotationsachse RT drehbar beweglich. Der Übertragungskörper 13 wird an einer Drehbewegung gemeinsam mit dem Antriebsglied 10 zwar nicht gehindert, durch seine Drehbewegbarkeit relativ zu dem Antriebsglied 10 können jedoch die durch die Antriebsbewegung des Antriebsglieds 10 verursachten, gegebenenfalls auf den Sensor 16, 17 wirkenden Reibungskräfte klein gehalten werden.

Der Übertragungskörper 13 bildet einen Deckel für das hülsenförmige Antriebsglied 10. Er ist in dem Hohlquerschnitt des Antriebsglieds 10 an dessen hinteren Ende aufgenommen. Für die Lagerung des Übertragungskörpers 13 ist in das hintere Ende des Antriebsglieds 10 eine Presshülse 11 eingesetzt, die den Übertragungskörper 13 axial linear führt und den Anschlag 12 bildet.

Die Figur zeigt die Fördereinrichtung in einem Zustand mit teilweise entleertem Reservoir 4.

In einem Ausgangszustand, in dem das Abtriebsglied 7 eine hintere Position einnimmt, drückt das Abtriebsglied 7 gegen den Übertragungskörper 13 und dieser gegen den Sensor 16, 17. Der Sensor 16, 17 erfährt hierdurch eine elastische Biegeverformung über die bei dem Kraftniveau "null" vorhandene Verformung hinaus, so dass der Sensor 16, 17 ein Ausgangssignal über die Leitung 18 ausgibt, das für die von dem Abtriebsglied 7 ausgeübte Druckkraft charakteristisch ist.

Die in einer Rückwärtsbewegung bei Erreichen der hinteren Position von dem Abtriebsglied 7 auf den Sensor 16, 17 ausgeübte Druckkraft, die das charakteristische Ausgangssignal bewirkt, kann so gewählt sein, dass solch eine Druckkraft im Normalbetrieb der Vorrichtung nicht auftritt, sondern allenfalls im Falle einer Okklusion in einem das Medikament führenden Teil des Geräts. Vorzugsweise entspricht das charakteristische Ausgangssignal jedoch einer Druckkraft, wie sie der Größe nach auch im Normalbetrieb bei der ungestörten Abtriebsbewegung des Abtriebsglieds 7 wegen dessen axialen Abstützung an dem Abtriebsglied 10 ebenfalls auftritt. Um das für die hintere Position des Abtriebsglieds 7 charakteristische Ausgangssignal von dem bei der üblichen Abtriebsbewegung auftretenden Ausgangssignal zu unterscheiden, nutzt die Überwachungseinrichtung, nämlich deren Antriebssteuerung 21 oder deren Ausgabeeinrichtung 22 oder eine andere Einrichtung der Überwachungseinrichtung, als Hilfsinformation die der Antriebssteuerung 21 bekannte Antriebsrichtung, im Ausführungsbeispiel Drehrichtung, des Antriebs 20. Aus der Größe des Ausgangssignals des Sensors 16, 17 und der Antriebsrichtungsinformation kann die Überwachungseinrichtung sicher unterscheiden, ob es sich bei dem über die Leitung 18 eingehenden Sensorsignal um ein Signal handelt, das dem Normalbetrieb der Fördereinrichtung während der Abtriebsbewegung des Abtriebsglieds 7 entspricht, oder um das charakteristische Ausgangssignal, das der Sensor 16, 17 erzeugt, wenn das Abtriebsglied 7 seine hintere Position erreicht.

Die für die Erfindung relevanten Abläufe bei einem Austausch des Reservoirs 4 werden nachfolgend anhand der Figur erläutert.

Es sei angenommen, dass sich die Fördereinrichtung in dem Ausgangszustand befindet, in dem das Abtriebsglied 7 entlang der Rotationsachse RT seine hintere Position einnimmt, in der es in und gegen die Vortriebsrichtung A aufgrund der mechanischen Kopplung mit dem Antriebsglied 10 und dessen mechanischer Kopplung mit dem Antrieb 20 festgesetzt ist. In diesem Ausgangszustand wird ein neues, gefülltes Reservoir 4 in die Aufnahme 3 entgegen der Vortriebsrichtung A eingesetzt. Durch das Einsetzen wird auch gleich die axial feste Verbindung zwischen dem Kolben 6 und dem Abtriebsglied 7 hergestellt. Nachdem das Reservoir 4 eingesetzt und die Aufnahme 3 am vorderen Ende verschlossen ist, werden die das Medikament führenden Teile des Geräts entlüftet, indem der Antrieb 20 das Antriebsglied 10 rotatorisch antreibt. Anschließend ist das Gerät bereit für die Verabreichung. Aufgrund der rotatorischen Antriebsbewegung des Antriebsglieds 10 in die Antriebsrichtung und die axiale Linearführung des Abtriebsglieds 7 durch die Verdrehsicherung 9 wird das Zwischenglied 8 entweder rein translatorisch in die Vortriebsrichtung A oder rein rotatorisch um die Rotationsachse RT oder sowohl translatorisch als auch rotatorisch angetrieben. Führt das Zwischenglied 8 eine Translationsbewegung in die Vortriebsrichtung A aus, nimmt es aufgrund des Gewindeeingriffs das Abtriebsglied 7 einfach mit, d. h. das Zwischenglied 8 und das Abtriebsglied 7 führen eine gemeinsame Abtriebsbewegung aus. Eine Rotationsbewegung des Zwischenglieds 8 bewirkt über den Gewindeeingriff mit dem Abtriebsglied 7 ebenfalls eine Abtriebsbewegung des Abtriebsglieds 7. Im Verlaufe seiner in die Vortriebsrichtung A gerichteten Abtriebsbewegung kann das Abtriebsglied 7 bis in seine vordere Endposition bewegt werden, in der das Zwischenglied 8 auf Anschlagkontakt gegen einen Anschlag des Abtriebsglieds 10 und das Abtriebsglied 7 auf Anschlagkontakt gegen einen Anschlag des Zwischenglieds 8 sind. In der vorderen Endposition des Abtriebsglieds 7 ist das Reservoir 4 entleert.

Während der Fördertätigkeit der Fördereinrichtung überwacht die Überwachungseinrichtung das Ausgangssignal des Sensors 16, 17 durch geeignete Vergleichsoperationen, beispielsweise durch Vergleich mit einem vorgegebenen, oberen und unteren Grenzwert, um frühzeitig das Auftreten einer Okklusion oder eines Lecks feststellen zu können. Der Vergleich wird in kurzen Zeitabständen periodisch durchgeführt, kann prinzipiell aber auch aperiodisch durchgeführt werden. Zusätzlich zu einem Vergleich mit fest vorgegebenen Grenzwerten oder auch statt dessen kann die Vergleichsoperation der Überwachungseinrichtung darin bestehen, die zu unterschiedlichen Zeitpunkten erhaltenen Ausgangssignale des Sensors 16, 17 miteinander zu vergleichen. Auch komplexere Vergleichsoperationen sind denkbar, beispielsweise der Vergleich von aufsummierten Signalen oder integrierten Signalen.

Für einen erneuten Austausch des Reservoirs 4 steuert die Antriebssteuerung 21 nach entsprechender Betätigung durch den Verwender den Antrieb 20 um. Der Antrieb 20 treibt das Antriebsglied 10 nun in die Gegenrichtung rotatorisch an. Diese der Antriebsbewegung bei Fördertätigkeit entgegengerichtete Antriebsbewegung des Antriebsglieds 10 bewirkt eine rein translatorische Rückwärtsbewegung des Abtriebsglieds 7 gegen die Vortriebsrichtung A. Im Zuge seiner Rückwärtsbewegung gelangt das Abtriebsglied 7 in Anschlagkontakt gegen den Übertragungskörper 13 und drückt diesen mit zunehmender Kraft gegen den Sensor 16, 17. Der Sensor 16, 17 gibt ein dieser Kraft entsprechendes Ausgangssignal an die Überwachungseinrichtung aus. Sobald die von dem Abtriebsglied 7 ausgeübte Kraft einen vorgegebenen Vergleichswert überschritten hat, was die Überwachungseinrichtung durch eine Vergleichsoperation feststellt, schaltet die Überwachungseinrichtung den Antrieb 20 ab. Gegebenenfalls wird das Erreichen der hinteren Position auch mittels der Ausgabeeinrichtung 20 dem Verwender zur Kenntnis gebracht, um dem Verwender beispielsweise anzuzeigen, dass der Sensor 16, 17 korrekt arbeitet.

### Bezugszeichen:

- 1: Gehäuse, Hüllstruktur
- 2: Gehäuse, Lagerstruktur
- 3: Aufnahme
- 4: Reservoir
- 5: Anschlag, Ringsteg, Gleitführung
- 6: Kolben
- 7: Abtriebsglied
- 8: Zwischenglied
- 9: Verdrehsicherung
- 10: Antriebsglied
- 11: Presshülse
- 12: Anschlag
- 13: Übertragungskörper
- 14: verzahnter Ringsteg
- 15: -
- 16: Sensor, Biegekörper
- 17: Sensor, Erhebung
- 18: Signalleitung
- 19: Getriebe
- 20: Antrieb
- 21: Antriebssteuerung
- 22: Ausgabeeinrichtung

## Patentansprüche

1. Vorrichtung zur Verabreichung eines Produkts, die Vorrichtung umfassend:
a) ein Gehäuse (1, 2) mit einem Produktreservoir (4) oder einer Aufnahme (3) für ein Produktreservoir (4),
b) ein Abtriebsglied (7), das eine Abtriebsbewegung in eine Vortriebsrichtung (A) ausführt, um Produkt aus dem Produktreservoir (4) auszuschütten, und das gegen die Vortriebsrichtung (A) eine Rückwärtsbewegung bis in eine hintere Position ausführen kann,
c) ein von dem Gehäuse (1, 2) zur Ausführung einer Antriebsbewegung bewegbar gelagertes Antriebsglied (10), das mit dem Abtriebsglied (7) so gekoppelt ist, dass die Antriebsbewegung die Abtriebsbewegung bewirkt und das Abtriebsglied (7) sich bei der Abtriebsbewegung an dem Antriebsglied (10) gegen die Vortriebsrichtung (A) abstützt,
d) eine Überwachungseinrichtung mit einer Ausgabeeinrichtung (22) zur Ausgabe eines Alarms und/oder einer Steuerung (21) für einen Antrieb (20) des Antriebsglieds (10)
e) und einen Sensor (16, 17), der bei der Rückwärtsbewegung des Abtriebsglieds (7) das Erreichen der hinteren Position detektiert und an die Überwachungseinrichtung ein für das Erreichen der hinteren Position charakteristisches Ausgangssignal ausgibt.

2. Vorrichtung nach Anspruch 1, **dadurch gekennzeichnet, dass** die Vorrichtung einen Antrieb (20) für das Antriebsglied (10) und eine Steuerung (21) für den Antrieb (20) umfasst und dass der Sensor (16, 17) das charakteristische Ausgangssignal an die Steuerung (21) ausgibt, wobei die Steuerung (21) den Antrieb (20) bei Empfang des charakteristischen Ausgangssignals festsetzt.

3. Vorrichtung nach einem der vorhergehenden Ansprüche, **dadurch gekennzeichnet, dass** der Sensor (16, 17) ein Berührungssensor ist und das Abtriebsglied (7) bei der Rückwärtsbewegung in Kontakt mit dem Sensor (16, 17) gelangt.

4. Vorrichtung nach dem vorhergehenden Anspruch, **dadurch gekennzeichnet, dass** der Sensor (16, 17) ein Kraftsensor ist, der durch die bei der Rückwärtsbewegung in der hinteren Position von dem Abtriebsglied (7) ausgeübten Kraft verformt wird.

5. Vorrichtung nach einem der zwei vorhergehenden Ansprüche, **dadurch gekennzeichnet, dass** das Abtriebsglied (7) durch die Rückwärtsbewegung in Kontakt mit dem Sensor (16, 17) gelangt und auf den Sensor (16, 17) eine Kraft ausübt, die zunimmt, sobald der Kontakt mit dem Sensor (16, 17) besteht.

6. Vorrichtung nach einem der vorhergehenden Ansprüche, **dadurch gekennzeichnet, dass** der Sensor (16, 17) eine Lagerkraft misst, die das Gehäuse (1, 2) aufnimmt, wenn das Abtriebsglied (7) die Abtriebsbewegung ausführt.

7. Vorrichtung nach einem der vorhergehenden Ansprüche, **dadurch gekennzeichnet, dass** das Antriebsglied (10) sich bei der Antriebsbewegung an dem Sensor (16, 17) abstützt.

8. Vorrichtung nach einem der vorhergehenden Ansprüche, **dadurch gekennzeichnet, dass** das Antriebsglied (10) in und gegen die Vortriebsrichtung (A) schwimmend gelagert ist und von dem Sensor (16, 17) elastisch abgestützt wird.

9. Vorrichtung nach einem der vorhergehenden Ansprüche, **dadurch gekennzeichnet, dass** der Sensor (16, 17) das Antriebsglied (10) gegen einen Anschlag (5) des Gehäuses (1, 2) drückt.

10. Vorrichtung nach einem der vorhergehenden Ansprüche, **dadurch gekennzeichnet, dass** das Antriebsglied (10) sich bei der Abtriebsbewegung des Abtriebsglieds (7) über einen relativ zu dem Antriebsglied (10) beweglichen Übertragungskörper (13) an dem Sensor (16, 17) abstützt, wobei der Sensor (16, 17) den Übertragungskörper (13) zumindest während der Abtriebsbewegung gegen einen Anschlag (12) des Antriebsglieds (10) drückt.

11. Vorrichtung nach einem der vorhergehenden Ansprüche, **dadurch gekennzeichnet, dass** das Antriebsglied (10) sich bei der Abtriebsbewegung des Abtriebsglieds (7) über einen relativ zu dem Antriebsglied (10) beweglichen Übertragungskörper (13) an dem Sensor (16, 17) abstützt und das Abtriebsglied (7) bei der Rückwärtsbewegung auf Anschlag gegen den Übertragungskörper (13) gelangt und den Übertragungskörper (13) in der hinteren Position gegen den Sensor (16, 17) drückt.

12. Vorrichtung nach einem der zwei vorhergehenden Ansprüche, **dadurch gekennzeichnet, dass** das Antriebsglied (10) relativ zu dem Übertragungskörper (13) drehbar ist.

13. Vorrichtung nach einem der drei vorhergehenden Ansprüche, **dadurch gekennzeichnet, dass** das Antriebsglied (10) den Übertragungskörper (13) in und gegen die Vortriebsrichtung (A) führt.

14. Vorrichtung nach einem der vier vorhergehenden Ansprüche, **dadurch gekennzeichnet, dass** das Antriebsglied (10) hülsenförmig ist und den Übertragungskörper (13) vorzugsweise umgibt.

15. Vorrichtung nach einem der vorhergehenden Ansprüche, **dadurch gekennzeichnet, dass** das Antriebsglied (10) um eine in die Vortriebsrichtung (A) weisende Rotationsachse (RT) drehbar gelagert und die Antriebsbewegung eine Drehbewegung um die Rotationsachse (RT) ist.

16. Vorrichtung nach einem der vorhergehenden Ansprüche, **dadurch gekennzeichnet, dass** das Antriebsglied (10) und das Abtriebsglied (7) Teleskopstufen einer die Ausschüttung des Produkts bewirkenden Fördereinrichtung sind.

17. Vorrichtung nach dem vorhergehenden Anspruch, **dadurch gekennzeichnet, dass** die Fördereinrichtung ein Zwischenglied (8) umfasst, das eine der Teleskopstufen bildet und mit dem Abtriebsglied (7) und dem Antriebsglied (10) so gekoppelt ist, dass die Antriebsbewegung des Antriebsglieds (10) eine translative Abtriebsbewegung des Abtriebsglieds (7) in die Vortriebsrichtung (A) alleine oder gemeinsam mit dem Zwischenglied (8) bewirkt.
